# EUROPEAN PATENT SPECIFICATION

(11) **EP 1 893 086 B1**
(45) Date of publication and mention of the grant of the patent: **17.04.2013**
(21) Application number: 05747584.0
(22) Date of filing: 24.05.2005
(51) Int. Cl.: A61B 5/11, A61B 5/00, A61B 5/103, A61N 1/36

(54) **A METHOD AND A MEDICAL DEVICE FOR EVALUATING THE PREVALENCE OF DIFFERENT POSTURES OF A PATIENT AND A COMPUTER READABLE MEDIUM FOR BRINGING A COMPUTER TO PERFORMING THE METHOD**
VERFAHREN UND MEDIZINPRODUKT ZUR BEWERTUNG DER PRÄVALENZ VON UNTERSCHIEDLICHEN HALTUNGEN EINES PATIENTEN UND COMPUTERLESBARES MEDIUM, DAS DEN COMPUTER DAZU BRINGT, DAS VERFAHREN DURCHZUFÜHREN
PROCEDE ET DISPOSITIF MEDICAL D'EVALUATION DE LA PREVALENCE DE DIFFERENTES POSTURES D'UN PATIENT ET SUPPORT LISIBLE PAR ORDINATEUR FAISANT EXECUTER LE PROCEDE A UN ORDINATEUR

(43) Date of publication of application: 05.03.2008
(73) Proprietor: St. Jude Medical AB, 175 84 Järfälla (SE)
(72) Inventor: HOLMSTRÖM, Nils, S-175 57 Järfälla (SE); ÖHLANDER, Malin, S-118 48 Stockholm (SE)
(74) Representative: Sjölander, Henrik
(86) International application number: PCT/SE2005/000777
(87) International publication number: WO 2006/126918

(56) References cited:
- US-A- 4 846 195
- US-A- 5 593 431
- US-A1- 2001 012 954
- US-A1- 2004 073 093
- US-A1- 2004 102 712
- US-B1- 6 473 640

## Description

### TECHNICAL FIELD

The present invention generally relates to implantable medical devices, such as cardiac-pacemakers and implantable cardioverter/defibrillators, and in particular to a method and medical device for evaluating the prevalence of different postures of a patient.

### BACKGROUND OF THE INVENTION

A severe problem associated with measurement of, inter alia, the blood pressure of the chambers, contractility, endocardial acceleration, blood flow, coronary blood flow, the sinus rate, the electrical bio-impedance, such as the thoracic impedance and the cardiogenic impedance is that the accurateness and reliability, and, hence the repeatability, of the obtained signals are greatly affected by factors like the body position of the patient, patient activity levels, heart rate, etc.. For example, it has been found that the body position of the patient is of major importance with regard to the blood pressure of the chambers, contractility, endocardial acceleration, blood flow, coronary blood flow, the sinus rate, the electrical bio-impedance, such as the thoracic impedance and the cardiogenic impedance, etc..

Repeatable measurements of such parameters are of a great value for identifying changes of many different conditions in the body of a patient. For example, electrical bio-impedance signals has been found to be an effective measure for identifying changes of many different conditions in the body of a patient, such as incipient pulmonary edema and the progression of pulmonary edema due to CHF, i.e. the accumulation of fluids in the lung-region associated with pulmonary edema affects the thoracic impedance, or more specifically the DC impedance level, since the resistivity of the lung changes in accordance with a change of the ratio of fluid to air. In addition to the thoracic impedance, the cardiogenic impedance, which is defined as the impedance or resistance variation that origins from cardiac contractions measured by electrodes inside or on the surface of the body, can be used for identifying changes of different conditions in the heart of a patient. For example, parameters such as the systolic and diastolic slopes, pre-ejection period and left ventricular ejection time indicative of different functions of the heart can be extracted from the cardiogenic impedance. The cardiogenic impedance variation correlates to the volume changes of the heart chambers, which can be used as an indication of the dynamic blood filling. Hence, changes of these parameters due to a change in the heart, for example, caused by a disease such as heart failure can be detected by monitoring or detecting changes of the cardiogenic impedance.

Furthermore, parameters associated with the heart, such as blood pressure of the chambers, contractility, endocardial acceleration, blood flow, coronary blood flow, the sinus rate etc., is very useful for diagnostic and/or therapeutic purposes, e.g. for identifying changes of different conditions in the heart of a patient. In order to be able to monitor changes of conditions of a patient, the measurements of the parameters, such blood pressure of the chambers, contractility, endocardial acceleration, blood flow, coronary blood flow, the electrical bio-impedance, such as the thoracic impedance and the cardiogenic impedance, must be substantially repeatable.

A number of attempts to eliminate or filter out error sources such as the body position of the patient, patient activity levels, heart rate frequency, etc have therefore been proposed. For example, U.S. Pat. No. 6,104,949 discloses a method and device for treatment of CHF, in which changes in the posture of the patient is correlated with changes of the trans-thoracic impedance. A posture sensing means indicates whether the patient lies down or is standing and the measurement of the trans-thoracic impedance is then correlated with periods when the patient is lying down. Thus, according to this known method, the obtaining of the impedance signals are correlated with periods when the patient is lying down.

However, it has recently been found that the posture or position dependence also is of a significant magnitude regarding different positions even when the patient is lying down, for example, whether the patient is lying on a side or is lying on the back. For example, regarding impedance measurements, a major reason is that the measurement depends on the measurement vector, i.e. the vector between the nodes that the current is applied between and the vector the voltage is measured between. When the body shifts position, these vectors will change since the gravity will influence, for example, tissue between the nodes and how it moves. Tests performed on animals have shown that the trans thoracic impedance may vary up to 20 % depending on which position the animal was lying in. Furthermore, in many applications there may also be desirable or even necessary to perform the measurements regularly at the same conditions and the time period.

Accordingly, there is a need of a method and medical device that are capable of evaluating the prevalence of different postures of a patient, which evaluation can be used to identify the most suitable posture for performing measurements in order to obtain measurements with a high degree of repeatability and accurateness.

### BRIEF DESCRIPTION OF THE INVENTION

Thus, an object of the present invention is to provide a method and medical device that are able to evaluate the prevalence of different postures of a patient.

Another object of the present invention is to provide a method and medical device that are able to obtain repeatable and accurate signals indicative of at least one physiological parameter.

These and other objects are achieved according to the present invention by providing a method, medical devices, and a computer readable medium having the features defined in the independent claim. Preferable embodiments of the invention are characterised by the dependent claims.

In the context of this application, the term "impedance" refers to the DC component of the impedance. The measured impedance consists of a DC component and an AC component, where the DC component is the baseline around which the AC component fluctuates. The DC component reflects the amount of tissue and fluids that are located between the measuring points that the impedance is measured in-between and the AC components reflects how respiration and cardiac activity influence the impedance signal.

For the purpose of clarity, the term "intra thoracic impedance" refers to an impedance measurement over the thorax by using an implantable medical device, i.e. an impedance measurement where the impedance measurement vector spans over the thorax.

Moreover, in order to clarify, the term "cardiogenic impedance" is defined as the impedance or resistance variation that origins from cardiac contractions or, in other words, the cardiac component of the impedance measured between electrodes in contact with the body.

According to an aspect of the present invention, there is provided a method for evaluating the prevalence of different postures of a patient according to claim 1.

According to a second aspect of the present invention, there is provided a medical device for evaluating the prevalence of different postures of a patient according to claim 11.

According to a third aspect of the present invention, there is provided a computer readable medium comprising instructions for bringing a computer to perform a method according to the first aspect.

Thus, the invention is based on the idea of recording patient posture signals during a predetermined patient monitoring period and using the history of the posture signals to determine the prevalence of the different postures of the patient.

This solution provides several advantages over the existing solutions. One advantage is that information regarding the posture pattern of a specific patient can be collected in an efficient and reliable way, which information, for example, may provide basis for a selection of specific posture as a triggering event for a measurement session of a posture sensitive physiological parameter.

According to the present invention, at least one measurement criterion is selected in dependence of the evaluation. For example, a set of specific signals suitable for a specific diagnostic purpose can be selected as measurement criterions, e.g. a specific position occurring at regular intervals. Accordingly, it is possible to customize the measurement criterions for a specific patient according to claim 11.

In the present invention, a measurement session in order to measure at least one physiological parameter is initiated when the selected at least one measurement criterion is satisfied. By performing the measurement only when the measurement criterion is satisfied, for example when the patient is in a specific posture, substantially repeatable signals can be obtained. Thereby, it is possible, for example, to monitor or detect changes of a condition of the patient or trends in the development of a condition of a patient in an effective way. Furthermore, this also entails that variations in the signals due to measurements in different body positions can be substantially eliminated, which is an evident risk with the method disclosed in, for example, U.S. Pat. No. 6,104,949, where the impedance measurements is correlated with moments when the patient is lying down and, therefore, the measurements are, in practical, performed in a number of different positions, i.e. when the patient is lying on either side or when the patient is lying on the back, etc. An additional advantage is that the measurements are initiated only when the predetermined measurement criterion is satisfied whereby a high efficiency with respect to current consumption is achieved.

According to an embodiment of the present invention, an activity level of the patient during patient monitoring period, it is determined whether the activity level is within at least one predetermined range, the information regarding the activity level range of the patient is stored together with each specific posture and the amount of time spent in each posture, and using the information regarding the activity level range of the patient in the evaluation by classifying the information with respect of specific postures, the amount of time spent in corresponding postures, and activity level range. Thereby, even more reliable measurements can be obtained since also the activity level of the patient is used to provide basis for a selection of a set of signals for the initiating of a measurement session.

The measurements are initiated in order to sense the intra thoracic impedance. Thereby, the progression of pulmonary edema can be monitored since the accumulation of fluids in the lung-region associated with pulmonary edema affects the thoracic impedance, or more specifically the DC impedance level, since the resistivity of the lung changes in accordance with a change of the ratio of fluid to air. The DC impedance level is negatively correlated with the amount of fluids in the lung. Due to the fact that pulmonary edema is a symptom of CHF, the development of CHF can be monitored indirectly by means of the intra thoracic impedance. For example, studies have shown that hospitalization due to the development of acute CHF with the symptom pulmonary edema was preceded two or three weeks by a drop in the DC impedance by approximately 10-15 %.

Further objects and advantages of the present invention will be discussed below by means of exemplifying embodiments.

### BRIEF DESCRIPTION OF THE DRAWINGS

In the following detailed description, reference will be made to the accompanying drawings, of which:
Fig. 1 is schematic diagram showing a medical device implanted in a patient in which device the present invention can be implemented.
Fig. 2 is block diagram of the primary functional components of a first embodiment of the medical device according to the present invention.
Fig. 3 is block diagram of the primary functional components of a second embodiment of the medical device according to the present invention.
Fig. 4 is a flow chart illustrating the steps in accordance with one embodiment of the present invention for evaluating the prevalence of different postures of a patient.
Figs. 5a and 5B show, during an eight hour period, the different positions of a patient and the amount of time the patient spends in each position and the corresponding position histogram for the information shown in Fig. 5a, respectively.
Fig. 6 is a flow chart illustrating the steps in accordance with one embodiment of the present invention for initiating a measurement session in order to measure a physiological parameter of a patient using the evaluation of the prevalence of the different postures of the patient.

### DETAILED DESCRIPTION OF THE INVENTION

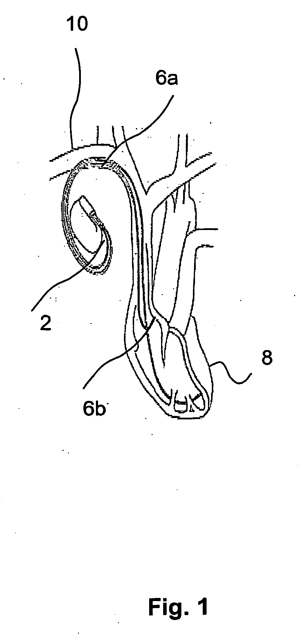

With reference to Fig. 1 there is shown a schematic diagram of a medical device implanted in a patient in which device the present invention can be implemented. As seen, this embodiment of the present invention is shown in the context of a pacemaker 2 implanted in a patient (not shown). The pacemaker 2 comprises a housing being hermetically sealed and biological inert. Normally, the housing is conductive and may, thus, serve as an electrode. One or more pacemaker leads, where only two are shown in Fig. 1 namely a ventricular lead 6a and an atrial lead 6b, are electrically coupled to the pacemaker 2 in a conventional manner. The leads 6a, 6b extend into the heart 8 via a vein 10 of the patient. One or more conductive electrodes for receiving electrical cardiac signals and/or for delivering electrical pacing to the heart 8 are arranged near the distal ends of the leads 6a, 6b. As the skilled man in the art realizes, the leads 6a, 6b may be implanted with its distal end located in either the atrium or ventricle of the heart 8.

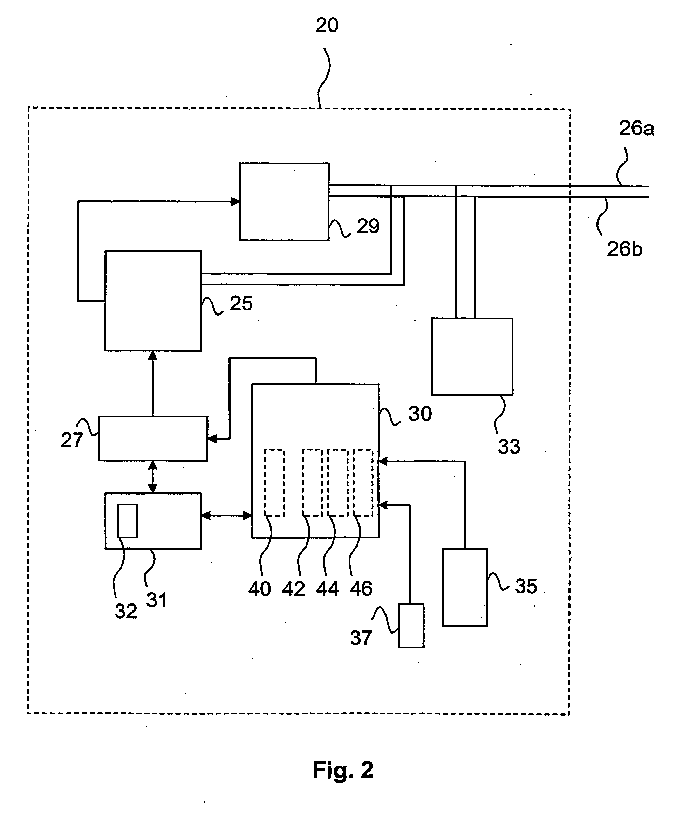

With reference now to Fig. 2, the configuration including the primary components of an embodiment of the present invention will be described. The illustrated embodiment comprises an implantable medical device 20, such as the pacemaker shown in Fig. 1, and leads 26a and 26b, of the same type as the leads 6a and 6b shown in Fig. 1, for delivering signals between the implantable medical device 20 and the patients heart. The leads 26a, 26b may be unipolar or bipolar, and may include any of the passive or active fixation means known in the art for fixation of the lead to the cardiac tissue. As an example, the lead distal tip (not shown) may include a tined tip or a fixation helix. The leads 26a, 26b comprises one or more electrodes (as described with reference to fig. 1), such a tip electrode or a ring electrode, arranged to, inter alia, transmit pacing pulses for causing depolarization of cardiac tissue adjacent to the electrode(-s) generated by a pace pulse generator 25 under influence of a control circuit 27. The control circuit 27 controls pace pulse parameters such as output voltage and pulse duration.

The control circuit 27 acts under influence of the microprocessor 30. A storing means 31 is connected to the control circuit 27 and the microprocessor 30, which storing means 31 may include a random access memory (RAM) and/or a non-volatile memory such as a read-only memory (ROM). In this embodiment, the storing means 31 comprises a computer program 32 comprising instructions for bringing a computer or a microprocessor to cause method steps in accordance with the present invention. Detected signals from the patients heart are processed in an input circuit 33 and are forwarded to the microprocessor 30 for use in logic timing determination in known manner. The implantable medical device 20 is powered by a battery 37, which supplies electrical power to all electrical active components of the medical device 20. Data contained in the storing means 31 can be transferred to a programmer (not shown) via a programmer interface (not shown) for use in analyzing system conditions, patient information, calculation of surrogate parameters such as systolic and diastolic slopes, the pre-ejection period, or left ventricular ejection time and changing pacing conditions, etc.

Furthermore, the implantable medical device 20 according to the present invention comprises position detecting sensor 35 arranged to detect the body position of a patient. In a preferred embodiment of the present invention, the position detecting sensor is a 3-dimensional orthogonal sensor arranged to sense whether the patient is, for example, standing or in a supine position, a left side position, a right side position, or a prone position. One example of such a 3-dimensional sensor is shown in US 6,044,297. Thereby, the position of the patient can be identified, for example, whether the patient is standing or resting in a supine position, a left side position, a right side position, or a prone position. The position detecting sensor 35 is connected to the microprocessor 30. Furthermore, the device 20 comprises determining means 40 arranged to determine specific body postures of the patient during a predetermined period of time using position signals from the position sensor, timing means 42 arranged to measure the amount of time the patient spends in each specific posture, and evaluating means 44 arranged to evaluate the prevalence of the different postures of the patient by classifying the obtained information with respect of specific postures and the amount of time spent in corresponding postures. The obtained information of the specific postures and amount of time spent in respective posture is stored in the storing means 31.

In a preferred embodiment, the determining means 40, the timing means 42 and the evaluating means 44 are integrated in the microprocessor 30 as being indicated in Fig. 2. The timing means 42 is also arranged to control the length of the monitoring period, i.e. the monitoring period can be set by means of the timing means 42. Moreover, the device comprises measurement criterion determining means 46 connected to the position detecting sensor 35. In this first embodiment, the measurement criterion determining means 46 is incorporated in the microprocessor 30, as indicated in Fig. 2.

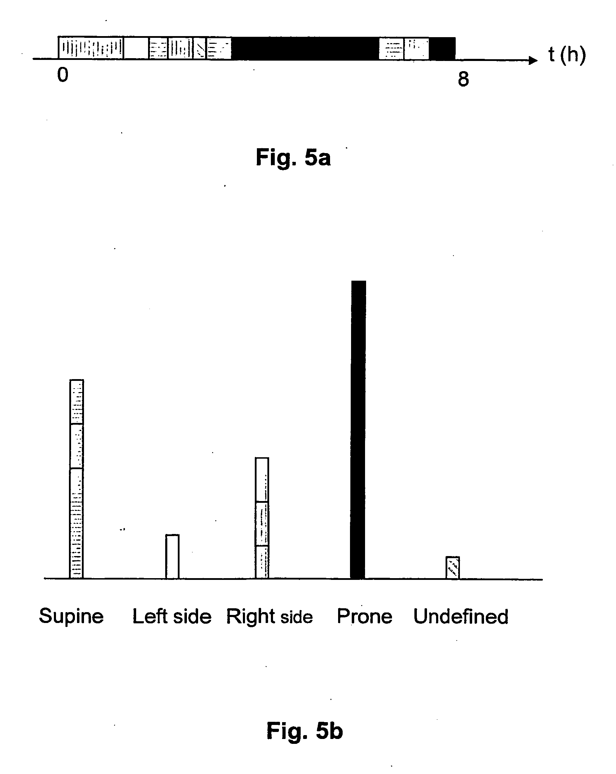

The evaluation means 44 is arranged to evaluate the prevalence of the different postures of the patient by classifying information with respect of specific postures and the amount of time spent in corresponding postures. For example, the evaluation means 44 can be arranged to make histograms showing the amount of time spent in each specific posture, see for example Figs. 5a and 5b, in accordance with conventional practice within the art. Of course, as the man skilled within the art realizes, other types of statistics can be made. This information can be used as a basis for selecting one or several measurement criterion, as will be discussed in more detail below. The evaluation means may be arranged to select one or several measurement criterions automatically, or the criterions may be selected manually by a doctor or medical attendant by means of the programmer communicating with the medical device 20, for example, via telemetry. Moreover, the resulting statistics, for example, the histogram or histograms can be transferred to the programmer for display on a screen.

The criterion determining means 46 is arranged to determine whether a set of measurement criterion is satisfied, which will be discussed in more detail below. If the set of criterion is satisfied, the criterion determining means 46 is arranged to apply a triggering signal to a measurement means 29, which is an impedance circuit 29 arranged to carry out impedance measurements. The measurement means 29 is arranged to, upon receiving the triggering signal, initiate an measurement session in order to obtain substantially repeatable signals to measure a physiological parameter.

The measurement means 29 is an impedance circuit 29 arranged to carry out impedance measurements. The impedance circuit is arranged to apply excitation current pulses between a first electrode arranged to positioned within a heart of the patient and second electrode in an embodiment where the intra thoracic impedance is measured. The impedance circuit 29 is also arranged to sense the impedance in the tissues between the first and second electrode to the excitation current pulse. Further, the impedance circuit 29 is coupled to the microprocessor 30, where processing of the obtained impedance signals can be performed. In an embodiment where the cardiac component of the electrical bio-impedance is sensed, the impedance circuit 29 is arranged to apply an excitation current pulse between a first electrode and a second electrode arranged to be positioned at different position within the heart of the patient and to sense the impedance in the tissues between the first and second electrode to the excitation current pulse. The microprocessor 30 may be arranged to extract the cardiac component of the sensed impedance. This cardiac component can be used for calculating parameters like systolic and diastolic slopes, the pre-ejection period, or left ventricular ejection time. This calculation can be performed in accordance with conventional practice within the art. The impedance sensing circuit 29 is controlled by the microprocessor 30 and the control circuit 27.

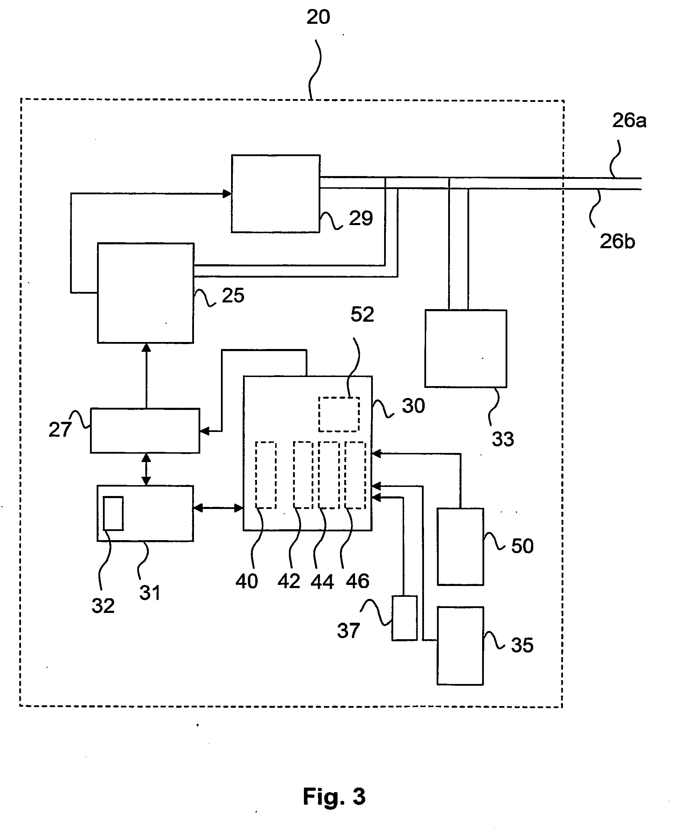

With reference now to Fig. 3, a second embodiment of the present invention will be discussed. Similar parts in the first and second embodiment will be denoted with the same reference numerals. Moreover, the description of like parts and the function of these will be omitted.

According to this embodiment, an activity sensor 50 is incorporated in the medical device in accordance with conventional practice within the art. An activity level determining means 52 is arranged to determine whether the sensed activity level is within at least one predetermined range. In this embodiment, the activity level determining means 52 is incorporated in the microprocessor 30. As will be discussed in further detail hereinafter, one or more activity level ranges may be used as measurement criterion in addition to, for example, the body posture. The storing means 31 is arranged to store information regarding the activity level range of the patient together with each specific posture and the amount of time spent in each posture; and the evaluating means 44 is arranged to evaluate the prevalence of the different postures of the patient by classifying the stored information with respect of specific postures, the amount of time spent in corresponding postures, and the activity level range.

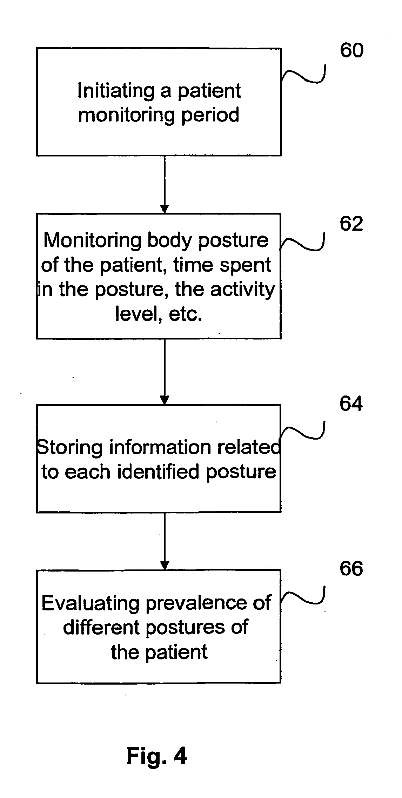

Referring now to Fig. 4, a high-level description of an embodiment of the method according to the present invention will be given. After an implantation of a medical device according to the present invention such as the first or second embodiment discussed above, a patient monitoring period can be executed in order to evaluate the prevalence of different postures of the patient, which evaluation, in turn, can be used to identify the most appropriate combination of signals for performing a sensor measurement session. This monitoring period can be initiated manually by the medical personal using a programmer communicating with the implanted device. The length of the period can be set manually and may last for e.g. 1 to 7 days. In operation, at step 60, the patient monitoring period is initiated in order to obtain information regarding the amount of time the patient spends in different positions at certain sets of criteria. Then, at step 62, the position sensor 35 monitors or detects the positions of the patient in order to detect the body posture of the patient, i.e. the sensor 35 is arranged to supply position indicating signals as described above during the patient monitoring period. The amount of time the patient spends in each position or posture is also measured. At step 64, information regarding each specific posture and the amount of time spent in each posture is stored. In another embodiment of the invention, an activity level sensor senses also the activity level of the patient. It is determined whether the activity level is within at least one predetermined activity level range, the information regarding the activity level range of the patient is stored together with each specific posture and the amount of time spent in each posture. According to a further embodiment of the present invention, also the heart rate of the patient is sensed and it is determined whether the heart rate is within a predetermined range, and the information regarding the heart rate is also stored in the storing means 31.

Thereafter, at step 66, when the patient monitoring period has ended, the prevalence of the different postures of the patient is evaluated by classifying the stored information with respect of specific postures and the amount of time spent in corresponding postures. Alternatively, this evaluation can be performed during the patient monitoring period. In the embodiment comprising an activity level sensor, also the activity level range is used in the evaluation. Furthermore, in the embodiment where the heart rate is sensed, also the heart rate level can be used in the evaluation. According to a preferred embodiment, the stored information is gathered in histograms as can be seen in Figs 5a and 5b. Fig. 5a shows, during an eight hour period, the different positions of a patient and the amount of time the patient spends in each position. Fig. 5b shows the corresponding position histogram comprising the information shown in Fig. 5a. The exemplifying results shown in Figs. 5a and 5b show that the patient during the monitored period of time spends most of the time sleeping on the tummy and least time sleeping on the left side. Moreover, it is shown that the supine position and the right side position are more evenly distributed over the measured period of time in comparison to, for example, prone. In the alternative embodiment of the present invention where the activity level of the patent is sensed, a histogram can be made for each activity level range. For example, one histogram showing the different postures when the patient is in a resting mode and one histogram showing the postures when the patient is in a non-resting mode. Of course, as the skilled man realizes, it is possible to use more than two activity level ranges.

According to the present invention, the above-mentioned evaluation is used as a base for selecting at least one measurement criterion. The selected measurement criterion is also memorized in the storing means 31 for use in measurements. That is, once the patient monitoring period is over, the histograms over the patients preferred body, or sensor, positions at different times of the day are analyzed in order to identify when it is a suitable point of time to measure a specific physiological parameter. There are however a number of conceivable parameters that may be taken into consideration when selecting the at least one measurement criterion:
- the specific physiological parameter to be measured;
- the total amount of time spent in a specific position;
- the number of periods of time spent in a specific position;
- the length of the periods of time spent in a specific position;
- the frequency of the periods of time spent in a specific position;
- the distribution of periods of time in a specific position over the total period of time.

It should be noted that this list is non-exhaustive, and that there are other parameters that also may be used in the selection of measurement criterion. For example, if the activity level of the patient is measured, the above mention parameters may be identified for a specific activity level range or a number of specific activity level ranges, e.g. when the patient is resting, when the patient is walking, and/or when the patient is exercising. This selection of measurement criterions may be performed automatically by the microprocessor in accordance with predefined rules. Such rules may be, for example, that the activity sensor indicates that a low body activity has been detected for a predetermined period, e.g. more than 5 minutes, and the position sensor indicates that the patient is supine. Accordingly, it is, for example, possible to reveal which body positions that are meaningful to use as criterion and which are not. If e.g. the patient never sleeps on the back it would be discovered in the evaluation and thus avoided as a measurement criterion. As an alternative, it is possible to perform the selection of measurement criterions manually by using of the programmer.

As mentioned above, the measurement criterion can be used for triggering a measurement session in order to measure a specific physiological parameter. That is, a measurement session is initiated each time the selected measurement criterion or set of criterions is satisfied, and, accordingly, there is possible to obtain substantially repeatable measurements. For example, if the physiological parameter of interest is to be measured when the patient is resting the measurement criterion, or in other words the preferred signal combination, can be when a low body activity has been detected for a predetermined period, e.g. more than 5 minutes, and the position sensor indicates that the patient is in supine. According to another example, the specific physiological parameter to be measured is the intra thoracic impedance. In this case, the measurement criteria may be that the patient is in a rest mode and is lying on his or hers back. In addition there is a number of further measurement conditions that can be used, e.g. that the measurement session is only initiated during a specific period of the day, that the heart rate level is within a specific range, that a specified period of time has elapsed since the preceding measurement session, etc.

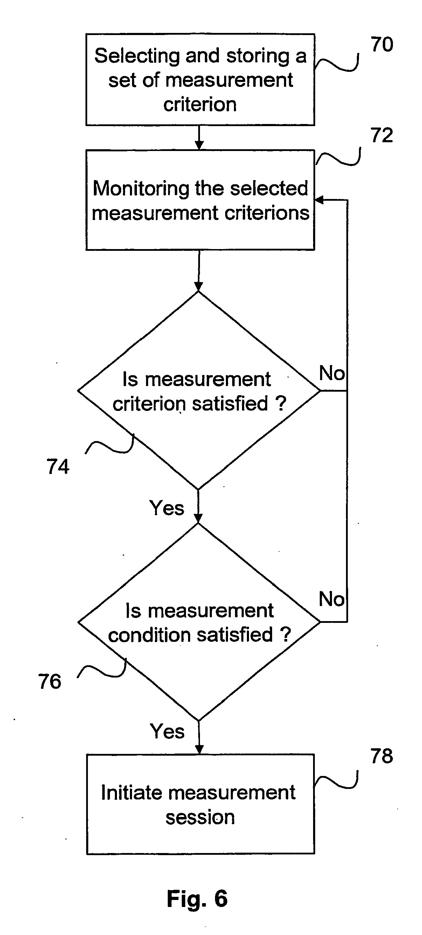

With reference now to Fig. 6, the procedure for performing measurements after the patient monitoring period has been completed according to one embodiment of the present invention will be discussed. This example is related to measurements of the intra thoracic impedance. There are a number of possible impedance configurations, i.e. ways of injecting current between two electrodes in the pacemaker and then to measure the voltage the current provokes between the electrodes. For example, impedance configurations can be uni-polar, bi-polar, tri-polar or quadro-polar. The configuration denominated as bi-polar means, in practice, a configuration where the current and the voltage is sent out and measured between the same two electrodes. When one of the electrodes used in a bi-polar measurement is the housing or the case, the configuration is called uni-polar. For example, in Fig. 1, between the housing of the pacemaker 2 and a right ventricular electrode arranged at the distal end of lead 6a. A tri-polar configuration uses three electrodes, i.e. the current injection and the voltage measurement share one electrode. As an example, the current can be sent out from the housing or the case of the medical device to a RV-tip and the voltage is measured between the case and RV-ring. In quadro-polar measurements, the current is sent out between electrodes and the voltage is measured between two entirely different electrodes, i.e. in this case there are four electrodes involved.

First, at step 70, at least one measurement criterion is selected and memorized in the storing means 31 in accordance with the discussion above. In this example, the selected measurement criterion is when the patient is lying on his or hers back. The measurement criterion can be selected automatically by the medical device or can be manually programmed by means of the programmer, and it is also possible to, for example, change the criterion if necessary. At step 72, the specific parameters of the set of measurement criterion are monitored, i.e., in this case the position sensor 35 monitors or detects the position of the patient in order to detect when the patient is in the predetermined position, i.e. when lying on the back. During periods when the patient is in other positions than the predetermined specific position, the impedance sensing circuit 29 is in an idle mode. Then, at step 74, it is checked whether the predetermined measurement criterion(-s) is or are satisfied. If the predetermined measurement criterion is satisfied, i.e. the patient is in the predetermined body position, it is checked, in step 76, whether additional measurement conditions in addition to the predetermined measurement criterion is or are satisfied, if any selected. In this case it is checked whether a specified period of time has elapsed since the preceding measurement session, for example, the condition may be that the sensing circuit is refractory during 1 hour after a valid measurement session. Otherwise, the procedure returns to step 72. Of course, the procedural steps 74 and 76 can be performed in one step as an alternative. If is determined that the additional measurements condition(-s) is (are) satisfied, the device proceeds to step 78, where the microprocessor 30 sends a triggering signal to the control circuit 27, which, in turn, puts the impedance sensing circuit 29 in an active mode where the sensing circuit 29 initiates an impedance sensing session, which may be performed in accordance with conventional practice.

Although an exemplary embodiment of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims.

## Claims

1. A method for evaluating the prevalence of different postures of a patient comprising the steps of
sensing (60) signals indicating the posture of said patient during a monitoring period having a predetermined length;
determining (62) specific body postures of the patient during said monitoring period using said signals;
measuring (62) the amount of time the patient spends in each of said specific postures; storing (64) information regarding each specific posture and the amount of time spent in each posture;
evaluating (66) the prevalence of said different postures of the patient by classifying said stored information with respect of specific postures and the amount of time spent in corresponding postures;
selecting (70) at least one measurement criterion in dependence of said evaluation; storing (70) said selected first measurement criterion
initiating (78) a measurement session in order to measure at least one physiological parameter when said selected at least one measurement criterion is satisfied,
wherein said at least one physiological parameter comprises an intra-thoracic impedance for monitoring a progression of pulmonary edema.

2. The method according to claim 1, wherein said at least one measurement criterion is a specific body posture of the patient.

3. The method according to any one of preceding claims, further comprising the steps of:
sensing an activity level of said patient during said monitoring period;
determining whether said activity level is within at least one predetermined range
storing information regarding the activity level range of the patient together with the information regarding each specific posture and the amount of time spent in each posture; and
using said information regarding the activity level range of the patient in said evaluation by classifying said information regarding the activity level range of the patient with respect of specific postures, the amount of time spent in corresponding postures, and activity level range.

4. The method according to claim 3, further comprising the step of selecting a second measurement criterion of said at least one measurement criterion, in dependence of said evaluation, for measurement of said least one specific physiological parameter; and storing said selected second measurement criterion.

5. The method according to claim 4, wherein said second measurement criterion is a specific activity level range of the patient.

6. The method according to any one of preceding claims, wherein the step of evaluating comprises the step of classifying said information with respect of distribution of said periods of time said patient spends in a specific posture over said monitoring period.

7. The method according to any one of preceding claims 2-6, further comprising the step of initiating said measurement session if said selected at least one measurement criterion is satisfied during a predetermined period of time of the day.

8. The method according to any one of preceding claims 1-7, further comprising the steps of:
sensing a heart rate of the patient;
determining whether the heart rate is within a predetermined range; and
selecting said predetermined heart rate range as a measurement criterion.

9. The method according to any one of preceding claims 2-8, further comprising the steps of:
at the initiation of an measurement session, measuring the amount of time elapsed since the preceding measurement session; and
cancelling the initiation of the measurement session if the amount of time elapsed since said preceding measurement session is within a predetermined range.

10. The method according to any one of preceding claims 2-9, further comprising the steps of:
storing the measurement result from said measurement sessions; and
making at least one histogram using said stored measurement results.

11. A medical device (20) for evaluating the prevalence of different postures of a patient comprising
a position sensing means (35) arranged to sense the posture of said patient; determining means (40) arranged to determine specific body postures of the patient during a monitoring period having a predetermined length using position signals from said position sensing means (35);
timing means (42) arranged to measure the amount of time the patient spends in each of said specific postures;
storing means (31) arranged to store information regarding each specific posture and the amount of time spent in each posture;
evaluating means (44) arranged to evaluate the prevalence of said different postures of the patient by classifying said stored information with respect of specific postures and the amount of time spent in corresponding postures;
wherein the storing means (31) is arranged to store at least one selected measurement criterion, said measurement criterion being selected in dependence of said evaluation
measurement means (29) arranged to execute a measurement session in order to measure a physiological parameter of said patient;
measurement criterion determining means (46) connected to said position sensing means (3 5) and said measurement means (29) and being arranged to determine whether said at least one measurement criterion is satisfied; and
if said at least one measurement criterion is satisfied, to apply a triggering signal to said measurement means (29), wherein said measurement means (29), upon receiving said triggering signal, initiates a measurement session, wherein said at least one physiological parameter comprises an intra-thoracic impedance for monitoring a progression of pulmonary edema.

12. The medical device according to claim 11, wherein said at least one measurement criterion is a specific body posture of the patient.

13. The medical device according to any one of preceding claims 11-12; further comprising:
activity level sensing means (50) arranged to sense an activity level of said patient;
activity level determining means (52) arranged to determine whether said sensed activity level is within at least one predetermined range;
wherein said storing means (31) is arranged to store information regarding the activity level range of the patient together with the information regarding each specific posture and the amount of time spent in each posture; and
wherein said evaluating means (44) is arranged to evaluate the prevalence of said different postures of the patient by classifying said stored information regarding the activity level range of the patient with respect of specific postures, the amount of time spent in corresponding postures, and the activity level range.

14. The medical device according to claims 13, wherein said storing means (31) is arranged to store a second measurement criterion of said at least one measurement criterion, said second measurement criterion being selected in dependence of said evaluation.

15. The medical device according to claim 14, wherein said second measurement criterion is a specific activity level range of the patient.

16. The medical device according to any one of preceding claims 11-15, wherein said evaluating means (44) is arranged to classify said information with respect of distribution of periods of time said patient spends in a specific posture over said predetermined patient monitoring period.

17. The medical device according to any one of preceding claims 12-16 wherein said measurement criterion determining means is arranged to apply said triggering signal to said measurement means if said selected at least one measurement criterion is satisfied during a predetermined period of time of the day.

18. The medical device according to any one of preceding claims 11-17, further comprising:
means for sensing a heart rate of the patient;
means for determining whether the heart rate is within a predetermined range; and
wherein said storing means is arranged to store said predetermined heart rate range as a measurement criterion.

19. The medical device according to any one of preceding claims 12-18, wherein:
said timing means (42) is arranged to, at the initiation of a measurement session, measure the amount of time elapsed since the preceding measurement session; and to apply a signal to said measurement means cancelling the initiation of the measurement session if the amount of time elapsed since said preceding measurement session is within a predetermined range.

20. The medical device according to any one of preceding claims 12-19, wherein said storing means (31) is arranged to store the measurement result from said measurement sessions; and wherein said evaluating means (44) is arranged to make at least one histogram using said stored measurement results.

21. A computer readable medium comprising instructions for bringing a computer to perform a method according to any one of the preceding claims 1-10.

## Patentansprüche

1. Verfahren zur Bewertung der Prävalenz von unterschiedlichen Haltungen eines Patienten, das folgende Schritte umfasst
Erfassen (60) von Signalen, die die Haltung des Patienten während eines Überwachungszeitraums mit einer festgelegten Länge angeben;
Ermitteln (62) von bestimmten Körperhaltungen des Patienten während des Überwachungszeitraums unter Verwendung der Signale;
Messen (62) der Zeitdauer, die der Patient in jeder der bestimmten Haltungen verbringt;
Speichern (64) von Informationen hinsichtlich jeder bestimmten Haltung und der Zeitdauer, die in jeder Haltung verbracht wird;
Bewerten (66) der Prävalenz der unterschiedlichen Haltungen des Patienten durch Einteilen der gespeicherten Informationen bezüglich bestimmter Haltungen und der Zeitdauer, die in entsprechenden Haltungen verbracht wird;
Auswählen (70) von mindestens einem Messkriterium in Abhängigkeit von der Bewertung;
Speichern (70) des ausgewählten ersten Messkriteriums
Beginnen (78) einer Messreihe zum Messen von mindestens einem physiologischen Parameter, wenn das ausgewählte mindestens eine Messkriterium erfüllt ist,
wobei der mindestens eine physiologische Parameter eine intrathorakale Impedanz zum Überwachen des Fortschritts eines Lungenödems umfasst.

2. Verfahren nach Anspruch 1, wobei es sich bei dem mindestens einen Messkriterium um eine bestimmte Körperhaltung des Patienten handelt.

3. Verfahren nach einem der vorhergehenden Ansprüche, das ferner folgende Schritte umfasst:
Erfassen eines Aktivitätsgrads des Patienten während des Überwachungszeitraums;
Ermitteln, ob der Aktivitätsgrad innerhalb von mindestens einem festgelegten Bereich liegt
Speichern von Informationen hinsichtlich des Aktivitätsgradbereichs des Patienten zusammen mit den Informationen hinsichtlich jeder bestimmten Haltung und der Zeitdauer, die in jeder Haltung verbracht wird; und
Verwenden der Informationen hinsichtlich des Aktivitätsgradbereichs des Patienten bei der Bewertung durch Einteilen der Informationen hinsichtlich des Aktivitätsgradbereichs des Patienten bezüglich bestimmter Haltungen, der Zeitdauer, die in entsprechenden Haltungen verbracht wird, und des Aktivitätsgradbereichs.

4. Verfahren nach Anspruch 3, das ferner den Schritt des Auswählens eines zweiten Messkriteriums von dem mindestens einen Messkriterium in Abhängigkeit von der Bewertung umfasst, zum Messen von dem mindestens einen bestimmten physiologischen Parameter; und des Speicherns des ausgewählten zweiten Messkriteriums.

5. Verfahren nach Anspruch 4, wobei es sich bei dem zweiten Messkriterium um einen bestimmten Aktivitätsgradbereich des Patienten handelt.

6. Verfahren nach einem der vorhergehenden Ansprüche, wobei der Schritt des Bewertens den Schritt des Einteilens der Informationen bezüglich der Verteilung der Zeiträume umfasst, die der Patient über den Überwachungszeitraum in einer bestimmten Haltung verbringt.

7. Verfahren nach einem der vorhergehenden Ansprüche 2 bis 6, das ferner den Schritt des Beginnens der Messreihe umfasst, wenn das ausgewählte mindestens eine Messkriterium während eines festgelegten Zeitraums des Tages erfüllt ist.

8. Verfahren nach einem der vorhergehenden Ansprüche 1 bis 7, das ferner folgende Schritte umfasst:
Erfassen einer Herzfrequenz des Patienten;
Ermitteln, ob die Herzfrequenz innerhalb von einem festgelegten Bereich liegt; und
Auswählen des festgelegten Herzfrequenzbereichs als Messkriterium.

9. Verfahren nach einem der vorhergehenden Ansprüche 2 bis 8, das ferner folgende Schritte umfasst:
beim Beginnen einer Messreihe Messen der Zeitdauer, die seit der vorhergehenden Messreihe vergangen ist; und
Abbrechen des Beginns der Messreihe, wenn die Zeitdauer, die seit der vorhergehenden Messreihe vergangen ist, in einem bestimmten Bereich liegt.

10. Verfahren nach einem der vorhergehenden Ansprüche 2 bis 9, das ferner folgende Schritte umfasst:
Speichern des Messergebnisses aus den Messreihen; und
Erstellen von mindestens einem Histogramm unter Verwendung der gespeicherten Messergebnisse.

11. Medizinprodukt (20) zur Bewertung der Prävalenz von unterschiedlichen Haltungen eines Patienten, umfassend
ein Positionserfassungsmittel (35), das so angeordnet ist, dass es die Haltung des Patienten erfasst;
ein Ermittlungsmittel (40), das so angeordnet ist, dass es bestimmte Körperhaltungen des Patienten während eines Überwachungszeitraums mit einer festgelegten Länge unter Verwendung von Positionssignalen von dem Positionserfassungsmittel (35) ermittelt;
ein Zeitmessmittel (42), das so angeordnet ist, dass es die Zeitdauer misst, die der Patient in jeder der bestimmten Haltungen verbringt;
ein Speichermittel (31), das so angeordnet ist, dass es Informationen hinsichtlich jeder bestimmten Haltung und der Zeitdauer speichert, die in jeder Haltung verbracht wird;
ein Bewertungsmittel (44), das so angeordnet ist, dass es die Prävalenz der unterschiedlichen Haltungen des Patienten durch Einteilen der gespeicherten Informationen bezüglich bestimmter Haltungen und der Zeitdauer, die in entsprechenden Haltungen verbracht wird, bewertet;
wobei das Speichermittel (31) so angeordnet ist, dass es mindestens ein ausgewähltes Messkriterium speichert, wobei das Messkriterium in Abhängigkeit von der Bewertung ausgewählt wird;
ein Messmittel (29), das so angeordnet ist, dass es eine Messreihe ausführt, um einen physiologischen Parameter des Patienten zu messen;
ein Messkriteriumermittlungsmittel (46), das mit dem Positionserfassungsmittel (35) und dem Messmittel (29) verbunden und so angeordnet ist, dass es ermittelt, ob das mindestens eine Messkriterium erfüllt ist; und
wenn das mindestens eine Messkriterium erfüllt ist, ein Auslösesignal an das Messmittel (29) anlegt, wobei das Messmittel (29), beim Empfangen des Auslösesignals, eine Messreihe beginnt;
wobei der mindestens eine physiologische Parameter eine intrathorakale Impedanz zum Überwachen des Fortschritts eines Lungenödems umfasst.

12. Medizinprodukt nach Anspruch 11, wobei es sich bei dem mindestens einen Messkriterium um eine bestimmte Körperhaltung des Patienten handelt.

13. Medizinprodukt nach einem der vorhergehenden Ansprüche 11 bis 12, ferner umfassend:
ein Aktivitätsgrad-Erfassungsmittel (50), das so angeordnet ist, dass es einen Aktivitätsgrad des Patienten erfasst;
ein Aktivitätsgradermittlungsmittel (52), das so angeordnet ist, dass es ermittelt, ob der erfasste Aktivitätsgrad innerhalb von mindestens einem festgelegten Bereich liegt;
wobei das Speichermittel (31) so angeordnet ist, dass es Informationen hinsichtlich des Aktivitätsgradbereichs des Patienten zusammen mit den Informationen hinsichtlich jeder bestimmten Haltung und der Zeitdauer speichert, die in jeder Haltung verbracht wird; und
wobei das Bewertungsmittel (44) so angeordnet ist, dass es die Prävalenz der unterschiedlichen Haltungen des Patienten durch Einteilen der gespeicherten Informationen hinsichtlich des Aktivitätsgradbereichs des Patienten bezüglich bestimmter Haltungen, der Zeitdauer, die in entsprechenden Haltungen verbracht wird, und des Aktivitätsgradbereichs bewertet.

14. Medizinprodukt nach Anspruch 13, wobei das Speichermittel (31) so angeordnet ist, dass es ein zweites Messkriterium von dem mindestens einen Messkriterium speichert, wobei das zweite Messkriterium in Abhängigkeit von der Bewertung ausgewählt wird.

15. Medizinprodukt nach Anspruch 14, wobei es sich bei dem zweiten Messkriterium um einen bestimmten Aktivitätsgradbereich des Patienten handelt.

16. Medizinprodukt nach einem der vorhergehenden Ansprüche 11 bis 15, wobei das Bewertungsmittel (44) so angeordnet ist, dass es die Informationen bezüglich der Verteilung von Zeiträumen einteilt, die der Patient über den bestimmten Patientenüberwachungszeitraum in einer bestimmten Haltung verbringt.

17. Medizinprodukt nach einem der vorhergehenden Ansprüche 12 bis 16, wobei das Messkriteriumermittlungsmittel so angeordnet ist, dass es das Auslösesignal an das Messmittel anlegt, wenn das ausgewählte mindestens eine Messkriterium während eines festgelegten Zeitraums des Tages erfüllt ist.

18. Medizinprodukt nach einem der vorhergehenden Ansprüche 11 bis 17, ferner umfassend:
Mittel zum Erfassen einer Herzfrequenz des Patienten;
Mittel zum Ermitteln, ob die Herzfrequenz innerhalb von einem festgelegten Bereich liegt; und
wobei das Speichermittel so angeordnet ist, dass es den festgelegten Herzfrequenzbereich als ein Messkriterium speichert.

19. Medizinprodukt nach einem der vorhergehenden Ansprüche 12 bis 18, wobei:
das Zeitmessmittel (42) so angeordnet ist, dass es bei Beginn einer Messreihe, die Zeitdauer misst, die seit der vorhergehenden Messreihe vergangen ist; und ein Signal an das Messmittel anlegt, das den Beginn der Messreihe abbricht, wenn die Zeitdauer, die seit der vorhergehenden Messreihe vergangen ist, in einem bestimmten Bereich liegt.

20. Medizinprodukt nach einem der vorhergehenden Ansprüche 12 bis 19, wobei das Speichermittel (31) so angeordnet ist, dass es das Messergebnis aus den Messreihen speichert; und wobei das Bewertungsmittel (44) so angeordnet ist, dass es mindestens ein Histogramm unter Verwendung der gespeicherten Messergebnisse erstellt.

21. Computerlesbares Medium, das Anweisungen enthält, um einen Computer dazu zu bringen, ein Verfahren nach einem der vorhergehenden Ansprüche 1 bis 10 durchzuführen.

## Revendications

1. Procédé d'évaluation de la prévalence de différentes postures d'un patient comprenant les étapes consistant à
détecter (60) des signaux indiquant la posture dudit patient pendant une période de surveillance présentant une longueur prédéterminée ;
déterminer (62) des postures corporelles spécifiques du patient pendant ladite période de surveillance en utilisant lesdits signaux ;
mesurer (62) la quantité de temps que le patient passe dans chacune desdites postures spécifiques ;
enregistrer (64) des informations concernant chaque posture spécifique et la quantité de temps passée dans chaque posture ;
évaluer (66) la prévalence desdites différentes postures du patient en classifiant lesdites informations enregistrées par rapport à des postures spécifiques et à la quantité de temps passée dans des postures correspondantes ;
sélectionner (70) au moins un critère de mesure en fonction de ladite évaluation ;
enregistrer (70) ledit premier critère de mesure sélectionné
lancer (78) une session de mesure afin de mesurer au moins un paramètre physiologique quand ledit au moins un critère de mesure sélectionné est satisfait,
dans lequel ledit au moins un paramètre physiologique comprend une impédance intrathoracique pour surveiller une progression d'un oedème pulmonaire.

2. Procédé selon la revendication 1, dans lequel ledit au moins un critère de mesure est une posture corporelle spécifique du patient.

3. Procédé selon l'une quelconque des revendications précédentes, comprenant en outre les étapes consistant à :
détecter un niveau d'activité dudit patient pendant ladite période de surveillance ;
déterminer si ledit niveau d'activité est situé dans au moins une plage prédéterminée
enregistrer des informations concernant la plage de niveau d'activité du patient avec les informations concernant chaque posture spécifique et la quantité de temps passée dans chaque posture ; et
utiliser lesdites informations concernant la plage de niveau d'activité du patient dans ladite évaluation en classifiant lesdites informations concernant la plage de niveau d'activité du patient par rapport à des postures spécifiques, à la quantité de temps passée dans des postures correspondantes, et à la plage de niveau d'activité.

4. Procédé selon la revendication 3, comprenant en outre l'étape consistant à sélectionner un second critère de mesure dudit au moins un critère de mesure en fonction de ladite évaluation, pour la mesure dudit au moins un paramètre physiologique spécifique, et enregistrer ledit second critère de mesure sélectionné.

5. Procédé selon la revendication 4, dans lequel ledit second critère de mesure est une plage de niveau d'activité spécifique du patient.

6. Procédé selon l'une quelconque des revendications précédentes, dans lequel l'étape d'évaluation comprend l'étape consistant à classifier lesdites informations par rapport à la distribution desdites périodes de temps que ledit patient passe dans une posture spécifique sur ladite période de surveillance.

7. Procédé selon l'une quelconque des revendications précédentes 2 à 6, comprenant en outre l'étape consistant à lancer ladite session de mesure si ledit au moins un critère de mesure sélectionné est satisfait pendant une période de temps prédéterminée de la journée.

8. Procédé selon l'une quelconque des revendications précédentes 1 à 7, comprenant en outre les étapes consistant à :
détecter une fréquence cardiaque du patient ;
déterminer si la fréquence cardiaque est située dans une plage prédéterminée ; et
sélectionner ladite plage de fréquence cardiaque prédéterminée en tant que critère de mesure.

9. Procédé selon l'une quelconque des revendications précédentes 2 à 8, comprenant en outre les étapes consistant à :
au lancement d'une session de mesure, mesurer la quantité de temps écoulée depuis la session de mesure précédente ; et
annuler le lancement de la session de mesure, si la quantité de temps écoulée depuis ladite session de mesure précédente est située dans une plage prédéterminée.

10. Procédé selon l'une quelconque des revendications précédentes 2 à 9, comprenant en outre les étapes consistant à :
enregistrer le résultat de mesure desdites sessions de mesure ; et
faire au moins un histogramme en utilisant lesdits résultats de mesure enregistrés.

11. Dispositif médical (20) pour évaluer la prévalence de différentes postures d'un patient comprenant
un moyen de détection de position (35) disposé pour détecter la posture dudit patient ;
un moyen de détermination (40) disposé pour déterminer des postures corporelles spécifiques du patient pendant une période de surveillance présentant une longueur prédéterminée en utilisant des signaux de position provenant dudit moyen de détection de position (35) ;
un moyen de chronométrage (42) disposé pour mesurer la quantité de temps que le patient passe dans chacune desdites postures spécifiques ;
un moyen d'enregistrement (31) disposé pour enregistrer des informations concernant chaque posture spécifique et la quantité de temps passée dans chaque posture ;
un moyen d'évaluation (44) disposé pour évaluer la prévalence desdites différentes postures du patient en classifiant lesdites informations enregistrées par rapport à des postures spécifiques et à la quantité de temps passée dans des postures correspondantes ; dans lequel le moyen d'enregistrement (31) est disposé pour enregistrer au moins un critère de mesure sélectionné, ledit critère de mesure étant sélectionné en fonction de ladite évaluation,
un moyen de mesure (29) disposé pour exécuter une session de mesure afin de mesurer un paramètre physiologique dudit patient ;
un moyen de détermination de critères de mesure (46) connecté audit moyen de détection de position (35) et audit moyen de mesure (29) et disposé pour déterminer si ledit au moins un critère de mesure est satisfait ; et
si ledit au moins un critère de mesure est satisfait, pour appliquer un signal de déclenchement audit moyen de mesure (29), dans lequel ledit moyen de mesure (29), lors de la réception dudit signal de déclenchement, lance une session de mesure, dans lequel ledit au moins un paramètre physiologique comprend une impédance intrathoracique pour surveiller une progression d'un oedème pulmonaire.

12. Dispositif médical selon la revendication 11, dans lequel ledit au moins un critère de mesure est une posture corporelle spécifique du patient.

13. Dispositif médical selon l'une quelconque des revendications précédentes 11 à 12 ; comprenant en outre :
un moyen de détection de niveau d'activité (50) disposé pour détecter un niveau d'activité dudit patient ;
un moyen de détermination de niveau d'activité (52) disposé pour déterminer si ledit niveau d'activité détecté est situé dans au moins une plage prédéterminée ;
dans lequel ledit moyen d'enregistrement (31) est disposé pour enregistrer des informations concernant la plage de niveau d'activité du patient avec les informations concernant chaque posture spécifique et la quantité de temps passée dans chaque posture ; et
dans lequel ledit moyen d'évaluation (44) est disposé pour évaluer la prévalence desdites différentes postures du patient en classifiant lesdites informations enregistrées concernant la plage de niveau d'activité du patient par rapport à des postures spécifiques, à la quantité de temps passée dans des postures correspondantes, et à la plage de niveau d'activité.

14. Dispositif médical selon la revendication 13, dans lequel ledit moyen d'enregistrement (31) est disposé pour enregistrer un second critère de mesure dudit au moins un critère de mesure, ledit second critère de mesure étant sélectionné en fonction de ladite évaluation.

15. Dispositif médical selon la revendication 14, dans lequel ledit second critère de mesure est une plage de niveau d'activité spécifique du patient.

16. Dispositif médical selon l'une quelconque des revendications précédentes 11 à 15, dans lequel ledit moyen d'évaluation (44) est disposé pour classifier lesdites informations par rapport à la distribution de périodes de temps que ledit patient passe dans une posture spécifique sur ladite période de surveillance prédéterminée du patient.

17. Dispositif médical selon l'une quelconque des revendications précédentes 12 à 16, dans lequel ledit moyen de détermination de critères de mesure est disposé pour appliquer ledit signal de déclenchement audit moyen de mesure si ledit au moins un critère de mesure sélectionné est satisfait pendant une période de temps prédéterminée de la journée.

18. Dispositif médical selon l'une quelconque des revendications précédentes 11 à 17 ; comprenant en outre :
un moyen pour détecter une fréquence cardiaque du patient ;
un moyen pour déterminer si la fréquence cardiaque est située dans une plage prédéterminée ; et
dans lequel ledit moyen d'enregistrement est disposé pour enregistrer ladite plage de fréquence cardiaque prédéterminée en tant que critère de mesure.

19. Dispositif médical selon l'une quelconque des revendications précédentes 12 à 18, dans lequel :
ledit moyen de chronométrage (42) est disposé pour, au lancement d'une session de mesure, mesurer la quantité de temps écoulée depuis la session de mesure précédente ; et pour appliquer un signal audit moyen de mesure annulant le lancement de la session de mesure si la quantité de temps écoulée depuis ladite session de mesure précédente est située dans une plage prédéterminée.

20. Dispositif médical selon l'une quelconque des revendications précédentes 12 à 19, dans lequel ledit moyen d'enregistrement (31) est disposé pour enregistrer le résultat de mesure desdites sessions de mesure ; et dans lequel ledit moyen d'évaluation (44) est disposé pour faire au moins un histogramme en utilisant lesdits résultats de mesure enregistrés.

21. Support lisible par ordinateur comprenant des instructions pour faire exécuter un procédé selon l'une quelconque des revendications précédentes 1 à 10 à un ordinateur.
